# EUROPEAN PATENT APPLICATION

(11) **EP 2 745 823 A1**
(43) Date of publication of application: **25.06.2014**
(21) Application number: 12198488.4
(22) Date of filing: 20.12.2012
(51) Int. Cl.: A61F 13/511

(54) **Absorbent article having a lotion composition and a method of making same**

(71) Applicant: The Procter & Gamble Company, Cincinnati, OH 45202 (US)
(72) Inventor: Gagliardi, Ivano, 65824 Schwalbach (DE); D'Addario, Roberto, 65824 Schwalbach (DE); Vandeloo, Uwe, 65824 Schwalbach (DE)
(74) Representative: Briatore, Andrea

(57) **Abstract**

The present invention relates to an absorbent article such as a diaper or sanitary napkin, and pantiliner. Specifically, the present invention relates to a lotion formulation which is applied on at least a portion of the body-facing surface of the topsheet in a specific manner. The present invention also provides a process for manufacturing an absorbent article comprising the lotion composition applied in a specific manner.

## Description

### FIELD OF THE INVENTION

The present invention relates to the field of absorbent articles such as a diaper, a pant, an adult incontinence product, an absorbent insert for a diaper or pant, a wipe or a feminine care absorbent article such as sanitary napkins or towels, and pantiliners. Specifically, the present invention relates to an absorbent article comprising a lotion composition which is applied on at least a portion of the body-facing surface of the topsheet in a specific manner.

### BACKGROUND OF THE INVENTION

Absorbent articles such as a diaper, or a feminine care absorbent article such as sanitary napkins and pantiliners are absorbent articles placed against or in proximity to a wearer's body. They are designed to absorb and contain exudates and body fluids. These absorbent articles typically include a topsheet, a backsheet and typically an absorbent structure positioned between the topsheet and the backsheet.

The topsheet of the absorbent articles come into contact with the wearer's skin. It can thus abrade the skin during use and cause skin irritation. In addition, the topsheet may not leave the skin of the wearer completely dry of body fluids. Exudates, menses and other vaginal secretions may damage the skin's outer barrier layer, which leads to skin inflammation and irritation, i.e. skin disorders.

In order to reduce the likelihood of skin disorders, many absorbent articles comprise a lotion composition which is applied on at least a portion of the body-facing surface of the topsheet.

U.S. Patent Application No. US 2001/025162 A1 refers to a disposable absorbent article having a lotion composition which is coated on the body-facing surface of the topsheet. The lotion composition is semi-solid or solid at ambient temperatures and is adapted to be transferred to the wearer's skin to improve skin health.

The lotion composition can be applied to the topsheet in different manners.

U.S. Patent No. 6,426,444 B2 relates to absorbent articles comprising a topsheet brought in contact with the skin of the wearer. The lotion composition is applied to the topsheet in the form of a non-uniform application of a lotion composition, e.g. the application of stripes that run in the longitudinal direction.

However, the transfer of the lotion composition from the body-facing surface of the topsheet to the skin of the wearer can still be improved.

Generally, the lotion composition is deposited on a large portion of the topsheet. But only a small percentage of the lotion composition applied to the topsheet is transferred to the wearer's skin. Therefore, the total amount of the lotion composition applied to the topsheet in order to obtain the desired percentage of the lotion composition transferred to the wearer's skin may be thus relatively high. This high amount of the lotion composition applied on the topsheet can be expensive and detrimental for fluid acquisition.

In addition, when the topsheet is almost completely covered with the lotion composition, the lotion composition may block the topsheet openings. The ability of the topsheet to transmit the bodily fluids to the underlying absorbent structure is thus reduced.

Hence, there is still a need to develop an absorbent article having a lotion composition which during use is transferred more effectively to the skin of the wearer.

There is also a need to develop an absorbent article having a lotion composition applied in a specific manner for which the performance of the absorbent article will not be affected, i.e. its capacity to absorb and contain the exudates and body fluids.

There is a need to develop a method to apply the lotion composition via the specific manner on the body-facing surface of the topsheet of the absorbent article which is economical and compatible with current high speed manufacturing lines.

### SUMMARY OF THE INVENTION

It is an object of the present invention to provide an absorbent article comprising: a backsheet; a topsheet joined to the backsheet, the topsheet defining a body-facing surface; a transverse centerline; a lotion composition deposited on at least a portion of the body-facing surface of the topsheet; **characterized in that** the lotion composition is applied to the body-facing surface of the topsheet in the form of a pattern of a plurality of at least two lotion stripes, the lotion stripes being separated by one or more lotion free areas; wherein each lotion free area is defined by the region between two neighboring lotion stripes; wherein the plurality of lotion stripes is oriented parallel with the transverse centerline; wherein the ratio between the width of each lotion free area and the width of any of the neighboring lotion stripes of each lotion free area is from 0.5 to 50.

In some embodiments, the present invention provides the absorbent article in the form of a pattern of a plurality of at least four lotion stripes being separated by at least three lotion free areas wherein the ratio between the width of the lotion free area and the width of any of the neighboring lotion stripes of each lotion free area is increasing from a transversal edge of the absorbent article towards the transversal centerline of the absorbent article.

The present invention also provides a method for applying the lotion composition on the body-facing surface of the absorbent article comprising the following steps:
(a) advancing in a production line, a plurality of absorbent articles towards a lotion depositing station, wherein each absorbent article has its transversal centerline parallel to the machine direction of the product manufacturing equipment;
(b) depositing a plurality of at least 2 lotion stripes having a lotion composition, the lotion stripes being separated by one or more lotion free areas wherein the plurality of lotion stripes is oriented parallel with the transverse centerline of the absorbent article, wherein the ratio between the width of the lotion free area and the width of any of the neighboring lotion stripes of each lotion free area is from 0.5 to 50.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is partially cut away perspective view of a pantiliner having a generally oval shaped absorbent structure;
Fig. 2 shows a topview of a pantiliner according to a first embodiment of the present invention;
Fig. 3 shows a topview of a pantiliner according to another embodiment of the present invention;
Fig. 4 shows a topview of a pantiliner according to another embodiment of the present invention;
Fig. 5 shows a schematic view of the manufacturing process.

### DETAILED DESCRIPTION OF THE INVENTION

### Definition of terms

The term "*absorbent article*" as used herein refers to devices which absorb and contain body exudates, and, more specifically, refers to devices which are placed against or in proximity to the body of the wearer to absorb and contain the various exudates discharged from the body. Typical absorbent articles of the present invention include but are not limited to diapers, adult incontinence briefs, training pants, diaper holders and liners, absorbent inserts and the like, as well as feminine care absorbent articles. Particularly, "*feminine care absorbent article*" as used herein refers to a personal care product used by women during menstruation, vaginal discharge, and other bodily functions related to the vulva. Feminine care absorbent article may include sanitary napkins or towels, interlabial absorbent articles, pantiliners.

Absorbent articles also include wipes, such as household cleaning wipes, baby wipes, and the like.

The term "*absorbent insert*" and "*insert*" as used herein refer to a component of a wearable absorbent article that is adapted to contain and/or absorb urine, feces, menses or any combination thereof, and is adapted to be installable and removable as a modular unit, from an outer cover or chassis of a diaper, a pant or diaper holder.

The term "*body-facing surface*" as used herein refers to surfaces of absorbent articles and/or their component materials which face the body of the wearer, while "*garment facing surface*" refers to the opposite surfaces of the absorbent articles and/or their component materials that face away from the wearer when the absorbent articles are worn. Absorbent articles and components thereof, including the topsheet, the backsheet, the absorbent structure, and any individual layers of their component materials, typically have a body-facing surface and a garment facing surface.

The term "*body*" as used herein refers to the outer layers formed by a mammalian epidermal tissues including the skin and the hair.

The term "*body fluid*" as used herein refers to any fluid produced by human body including, but not limited to perspiration, urine, menstrual fluids, vaginal secretions and the like.

"*Comprise*," "*comprising*," and "*comprises*" as used herein are open ended terms, each specifying the presence of what follows, e.g., a component, but not precluding the presence of other features, e.g., elements, steps or components known in the art, or disclosed herein.

The term "*Cross Machine Direction*" or "*cross direction*" or "*CD*" as used herein means the direction perpendicular to the machine direction in the same plane of the endless sheet of absorbent article.

The term "*effective amount*" as used herein refers to an amount of the lotion composition used in the present invention, which, when applied to the topsheet of the absorbent article, will be effective for promoting the skin health of the wearer.

The term "*exudate*" as used herein refers to material from a source internal to the body, such as urine, menses, feces and mucus.

The term "*longitudinal*" as used herein refers to a line, axis or direction in the plane of the absorbent article that is generally aligned with (e.g., approximately parallel to) a vertical plane which bisects a standing wearer into left and right body halves when the absorbent article is worn.

The term "*lotion composition*" as used herein refers to a composition which is applied to the topsheet of the absorbent article. The lotion composition is transferable to the skin of the wearer to deliver skin benefits, e.g. moisturizing, lubificating, for reducing any skin rash. The lotion composition may comprise a carrier such as water and active ingredients including skin treatment agents and/or probiotics, prebiotics, fungicides, bactericides, phytoestrogens, pH modifiers and the like. Additional optional ingredients may be added to the lotion as desired, as described herein, to form a lotion composition. The lotion may form a film on the surface of the skin and may provide increased repellency of residual soils or exudates or improved wearability of the absorbent article, along with improving the health of the skin.

The term "*lotion stripe*" as used herein again includes the lotion composition but is not limited to a rectangular shape, a dog-bone shape, an hourglass shape, and a triangular shape.

The term "*machine direction*" or "*MD*" as used herein means the direction parallel to the flow of the endless sheet of absorbent articles through the absorbent article making machine and/or product manufacturing equipment.

The terms "*migrate*", "*migration*", or "*migrating*" mean the lotion composition moves from one place to another place by way of movement on a material or permeation through an intervening material.

The term "*immobilizing agent*" as used herein refers to an agent capable of immobilizing the lotion composition in the desired location in or on a substrate. The immobilizing agent counteracts the tendency of the lotion composition to migrate or flow by keeping the lotion composition primarily localized on the surface or in the region of the substrate to which the lotion composition is applied.

The term "*pantiliner*" or "*panty-liner*" as used herein refers to an absorbent article that is less bulky than a sanitary napkin which is generally worn by women between their menstrual periods.

The term "*sanitary napkin*" as used herein refers to an absorbent article which is worn by females adjacent to the pudendal region that is intended to absorb and contain the various exudates which are discharged from the body.

The term "*skin disorders*" as used herein refers to symptoms associated with irritating, acute, or chronic skin abnormalities. Examples of such symptoms include, but are not limited to, itching, inflammation, rash, burning, stinging, redness, swelling, sensitivity, sensation of heat, flaking/scaling, malodor, and the like.

The term "*skin treatment agent*" as used herein to include treatments effective on skin or hair of mammalian bodies, and refers to materials that when transferred to the body are capable of preventing, reducing, and/or eliminating occurrences of skin disorders, particularly skin disorders associated with erythema, malodor, and bacterial infections.

The term "*semisolid*" is meant that the lotion composition has a rheology typical of pseudoplastic or plastic fluids. When no shear is applied, the lotions compositions can have the appearance of a semi-solid but can be made to flow as the shear rate is increased. This is due to the fact that while the lotion composition contains primarily solid components, it also includes some minor liquid components.

The terms "*transfer*" or "*transferable*" as used herein, when used in the context of the lotion composition, refers to the lotion composition moving from one area of the absorbent article to the skin of the wearer or to another area on the absorbent article not by way of migration but by way of direct contact with the lotion composition, such as in a blotting effect.

The term "*transverse*" or "*lateral*" as used herein, are interchangeable, and refers to a line, axis or direction which lies in the plane of the absorbent article that is generally perpendicular to the longitudinal direction.

### Absorbent article and feminine care absorbent article

An absorbent article refers to devices which absorb and contain body exudates, and, more specifically, refers to devices which are placed against or in proximity to the body of the wearer to absorb and contain the various exudates discharged from the body. Typical absorbent articles of the present invention include but are not limited to diapers, adult incontinence briefs, training pants, diaper holders and liners, absorbent inserts and the like, as well as feminine care absorbent articles.

A feminine care absorbent article is a personal care product used by women during menstruation to absorb and retain menses, vaginal discharge, and other bodily functions related to vulva. The feminine care absorbent article may be disposable, which refers to a device which is not intended to be laundered or otherwise restored or reused as an absorbent article after a single use.

In the present invention, the feminine care absorbent article comprises at least a backsheet, a topsheet which is joined to the backsheet, and typically an absorbent structure. The topsheet has a body-facing surface. A lotion composition is applied to the body-facing surface of the topsheet in a specific manner, which results in increased effectiveness for the transfer of the lotion composition to the wearer's skin.

The application patterns of the lotion composition according to the present invention will be now discussed with reference to a pantiliner. However, the present invention is clearly not limited to the use of the pantiliner and the lotion composition can be applied using the same specific application patterns on a topsheet of any absorbent articles such as a diaper or a sanitary napkin.

### Pantiliner

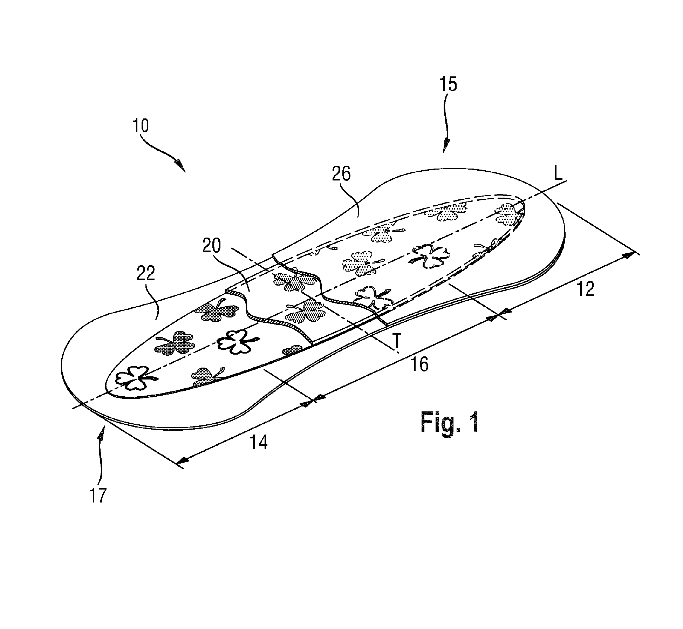

An exemplary pantiliner 10 is shown in partially cut-away perspective view in Fig. 1. The pantiliner 10 has two end regions 12 and 14 and a middle region 16 (i.e. a crotch region). The pantiliner 10 has a body-facing surface 15 and a garment facing side 17.

The exemplary pantiliner 10 represented in Fig. 1 comprises the following layers: a backsheet 22, a topsheet 26 which is joined to the backsheet 22, and an absorbent structure 20. The "*top*" of the absorbent article is defined herein as the body-facing surface 15 when the liner is in use, the "*bottom*" is the opposite surface of the absorbent article, i.e. the garment facing surface 17.

The backsheet 22 can be any backsheet material known in the art, including e.g. polymeric films, breathable films and nonwoven materials.

The absorbent structure 20 is typically positioned between the topsheet 26 and the backsheet 22. The size and shape of the absorbent structure 20 can be altered to meet absorbent capacity requirements, and to provide comfort to the wearer. The absorbent structure 20 suitable for use in the present invention can be any liquid-absorbent material known in the art for use in absorbent articles.

While the pantiliner 10 may have any shape known in the art, a typical shape is generally "*hourglass*" shaped. Transverse width is generally defined as the dimension perpendicular to the dimension, which is defined as length, running from end region 12 to end region 14 along a longitudinal centerline L. A transverse centerline T is the line perpendicular to the longitudinal centerline L at the middle of the pantiliner 10. The pantiliner 10 is at least symmetrical along the longitudinal centerline.

The garment facing side 17 can have thereon pressure sensitive adhesive for affixing to a wearer's undergarments. Typically, such adhesive is covered with a release strip which is removed before affixing to the undergarment.

Pantiliners can also be provided with lateral extensions known commonly in the art as "*flaps*" or "*wings*" (not shown) intended to extend over and cover the panty elastics in the crotch region of the user's undergarment. However, wings are normally not used with pantiliners.

Likewise, the topsheet 26 of the pantiliner 10 can have various optional characteristics, as it is known in the art. For example, the topsheet 26 can have channels embossed therein to direct fluid flow, and can have apertures there through to aid in fluid acquisition.

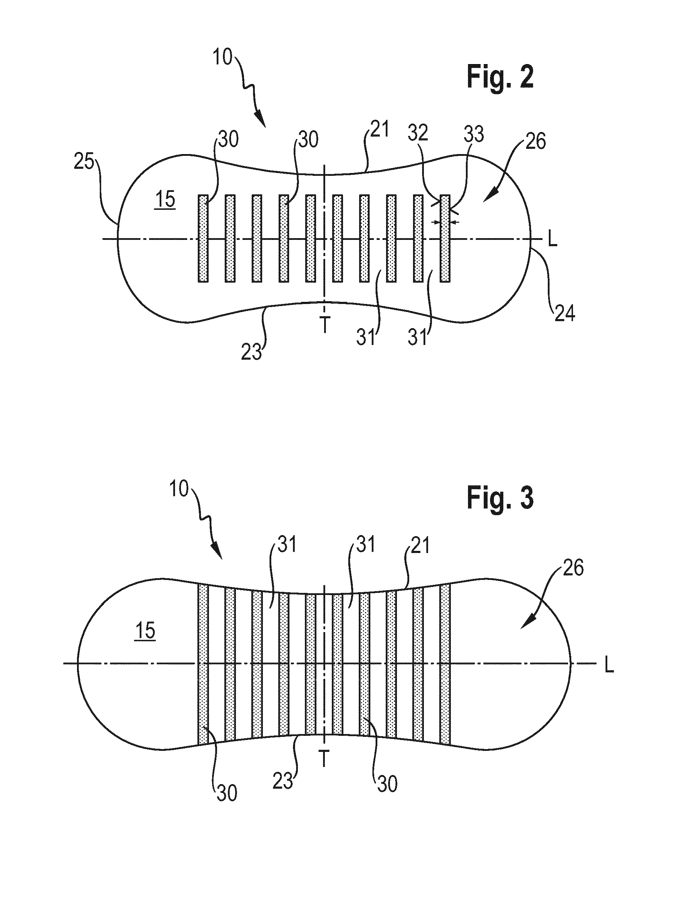

The topsheet 26 of the pantiliner 10 of the present invention has a lotion composition applied to the body-facing surface 15 in the form of a plurality of at least two lotion stripes 30 (e.g. Fig. 2).

### Absorbent article having a lotion composition applied in a specific manner

According to the present invention, the lotion composition as described above has been applied in a stripe pattern to the topsheet of the absorbent article, e.g. the pantiliner 10.

Fig. 2 is a top view of the pantiliner 10 according to a first embodiment of the present invention.

The lotion composition is applied to the body-facing surface 15 of the topsheet 26 in the form of a pattern of a plurality of at least two lotion stripes 30. Each lotion free area 31 is defined by the region between two neighboring lotion stripes 30.

Each lotion stripe 30 may be defined by two transversal edges 32, 33 parallel to the transverse centerline of the pantiliner 10. The width of each lotion stripe 30 is extending parallel with the longitudinal centerline and is the distance from one of its transversal edges 32 to the other transversal edge 33. The width of each lotion stripe 30 is measured along the longitudinal centerline. The width of each lotion stripe 30 may be from about 2 mm to about 25 mm or from about 3 mm to about 10 mm or from about 3 mm to about 6 mm.

The pantiliner 10 has two longitudinal edges 21, 23 and two transversal edges 24, 25. Each lotion stripe 30 may extend along the entire transverse centerline of the absorbent article 10 until reaching the longitudinal edges 21, 23 of the absorbent article 10 (Fig. 3), or can be shorter and centered along and around the transverse centerline (Fig. 2).

Similarly, each lotion free area 31 is a stripe which does not contain any lotion composition, i.e. the free space between two neighboring lotion stripes 30. The width of each lotion free area 31 is also extending parallel with the longitudinal centerline. The width of each lotion free area 31 is the distance between one transversal edge of one lotion stripe 30 to the immediate neighboring transversal edge of the next neighboring lotion stripe 30. The width of each lotion free area 31 is measured along the longitudinal centerline.

Each lotion stripe 30 may be arranged in a pattern as shown in Fig. 2 where: All lotion stripes 30 have the same width and all lotion stripes 30 are separated by lotion free areas 31 which have also the same width. Alternatively, each lotion stripe 30 and each lotion free area 31 may have different widths. The lotion stripes 30 may be of equal width whereas the lotion free areas 31 separating the lotion stripes 30 may be not of equal width.

In all embodiments of the present invention, the width of each lotion free area 31 is from about 0.25 mm to about 80 mm or from about 3 mm to about 40 mm or from about 3 mm to about 15 mm.

The plurality of lotion stripes 30 are oriented parallel with the transverse centerline of the absorbent article 10.

This specific arrangement has been found to provide a better transfer of the lotion composition to the skin of the wearer, when it is compared to prior art arrangements where the plurality of the lotion stripes 30 is oriented parallel with the longitudinal centerline.

Without being bound by theory, it is believed that this improved transfer is promoted by the movement of the pelvis of the wearer. Indeed, the wearer's pelvis essentially moves in a direction parallel with the longitudinal centerline of the absorbent article 10. Due to the contact of the body-facing surface 15 of the topsheet 26 with the skin of the wearer, and also due to the movement of the pelvis imposed to the absorbent article 10, each lotion stripe 30 is spreading on both neighboring sides, i.e. the two neighboring lotion free areas 31 of each lotion stripe 30. This results in an increase of the surface area of the skin which is in contact with the lotion composition. A higher percentage of the lotion composition applied to the topsheet 26 is therefore transferred to the wearer's skin although the total amount of the lotion composition applied to the absorbent article 10 may be reduced.

The ratio between the width of each lotion free area 31 and the width of any of the neighboring lotion stripes 30 of each lotion free area 31 is at least from about 0.5 to about 50 or from about 1.0 to about 50 or from about 1.2 to about 50 or from about 1.2 to about 20 or from about 1.2 to about 10 or from at least 1.2 to 5.

Without being bound by theory, the more the width of each lotion free area 31 is increasing relatively to each neighboring lotion stripe 30, the more each lotion stripe 30 has space to spread on the body-facing surface 15 of the topsheet 26. Hence, the lotion composition is more efficiently transferred to a larger area of the wearer's skin to provide all the skin benefits to reduce the skin disorders.

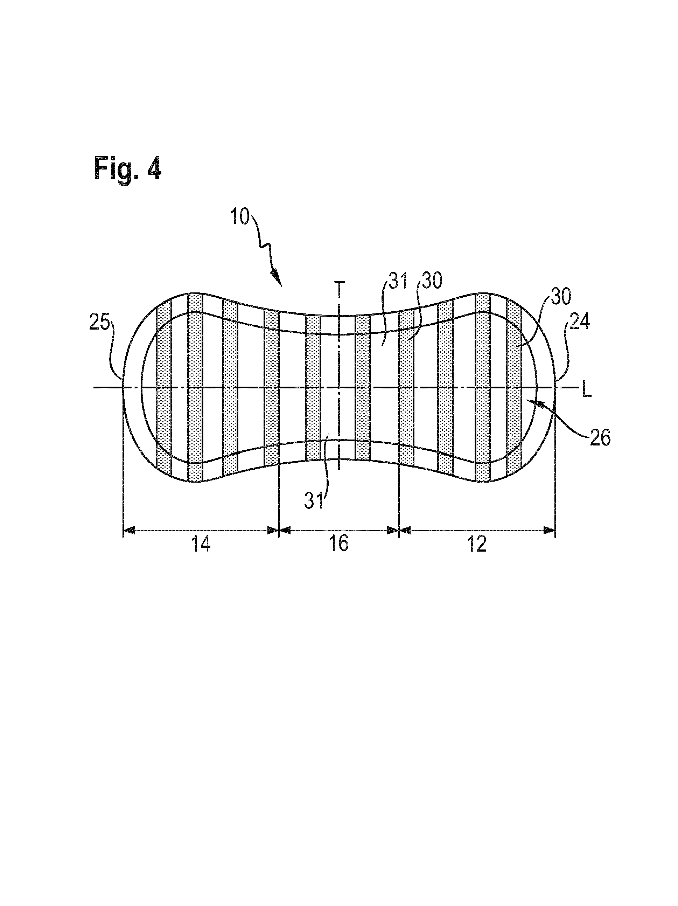

Fig. 4 is a top view of a pantiliner 10 according to another embodiment of the present invention.

In this embodiment, each lotion stripe 30 of the plurality of lotion stripes 30 is also oriented parallel with the transverse centerline over the full width of the absorbent article 10. However, the lotion free area 31 at the crotch region 16 of the pantiliner 10 is wider than the lotion free areas 31 in proximity of the transversal edges 24, 25 of the absorbent article 10.

In particular, the lotion composition is applied in the form of a pattern of a plurality of at least four lotion stripes 30 which are separated by at least three lotion free areas 31. The ratio between the width of the lotion free area 31 and the width of any of the neighboring lotion stripes 30 of each lotion free area 31 may increase from a transversal edge of the absorbent article 10 towards the transversal centerline of the absorbent article 10.

This embodiment is particularly advantageous because the body fluids are generally discharged at the center of the absorbent article 10, i.e. the crotch region 16. Lotion compositions useful in the present invention typically exhibit a hydrophobic character. Lotion compositions may hinder the acquisition and the absorption of the body fluids by the absorbent structure 20. Hence, with a wider lotion free area 31 at the crotch region 16, it is avoided that a lotion composition may impart the capacity of the absorbent article 10 to promptly absorb fluids in the insult region.

Generally, a safe and effective amount of the lotion composition is applied to the absorbent article 10 described herein wherein such safe and effective amounts include applying from about 5 mg to about 100 mg or from about 10 mg to about 50 mg or from about 15 mg to about 45 mg of the lotion composition to the absorbent article.

Particularly, each lotion stripe 30 applied on the body-facing surface 15 of the topsheet 26 of the absorbent article 10 may comprise from about 5 g/m² to about 30 g/m² or preferably from about 5 g/m² to about 20 g/m² or from about 10 g/m² to about 20 g/m² of the lotion composition.

The safe and effective amount of the lotion composition that will transfer or migrate to the body will depend on factors such as the type of lotion composition that is applied, the portion of the body-facing surface 15 where the lotion composition is applied, and the type of absorbent article 10 used to administer the lotion composition.

Any suitable method can be used in determining the amount of a lotion composition described herein that is transferred to the body of a wearer during use of the absorbent article 10 containing the lotion composition. An example of specific methods for the calculation of transfer amounts of lotion compositions include Gas Chromatographic and other quantitative analytical procedures that involve the analysis of *in vivo* skin analog materials. A suitable Gas Chromatographic procedure is more fully described in WO 99/45973, Donald C. Roe et al, published September 16, 1999.

### Lotion composition

At least a portion of the body-facing surface 15 of the topsheet 26 comprises an effective amount of the lotion composition. Typically, a suitable lotion composition is semi-solid or solid at about 25°C and which is partially transferable to the wearer's skin. In certain embodiments, the lotion composition may comprise from about 60 to about 99.9% of a carrier comprising a petroleum based hydrocarbon and lower molecular weight glycols or polyols and from about 0.1 to about 40% of a fatty alcohol with a melting point from about 45°C to about 110°C.

The lotion compositions of the present invention may also comprise a combination of skin treatment agents for the skin such as hexamidine, zinc oxide, and niacinamide which are highly effective in the prevention and treatment of erythema, malodor, and bacterial skin disorders, especially when these lotion compositions are administered to the skin from application on absorbent articles.

The lotion compositions may also comprise other active ingredients beneficial for the wearer's skin, e.g. probiotics, prebiotics, fungicides, bactericides, phytoestrogens, pH modifiers and the like.

Lotion compositions usable in the present invention are detailed below and are described in the International Application No. WO 2007/029198 A2.

### I. Skin Treatment Agents

The lotion compositions of the present invention may comprise relatively low concentrations of a combination of skin treatment agents that are capable of reducing and eliminating the occurrence of skin disorders. Skin disorders can result from contact between the skin and moisture-laden air. Skin disorders may result from prolonged moist human tissue that can occur from the skin being exposed to moisture or other body exudates. Skin disorders may also be generated from contact between the skin and microbial or bacterial agents. Skin treatments agents may be for instance hexamidine, zinc oxide, niacinamine or a combination of these agents, e.g. hexamidine, zinc oxide, and niacinamide or hexamadine and zinc oxide or hexamadine and niacinamide.

### A. Hexamidine

The lotion compositions of the present invention may comprise hexamidine skin treatment agent at concentrations ranging from about 0.001 % to about 0.1 % or from about 0.005% to about 0.1%, or from about 0.01 % to about 0.1 % by weight of the lotion composition. The hexamidine skin treatment agent suitable for use herein has the following formula:

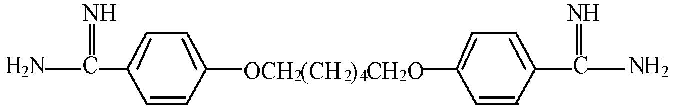

### B. Zinc oxide (ZnO)

The lotion compositions of the present invention may comprise zinc oxide skin treatment agent at concentrations ranging from about 0.001% to about 10% or from about 0.005% to about 5% or from about 0.005% to about 2%, most preferably from about 0.01 % to about 1% by weight of the lotion composition. The zinc oxide skin treatment agent may be included in the compositions, provided that the zinc oxide can readily combine with the hexamidine and niacinamide skin treatment agents to provide antimicrobial benefits.

### C. Niacimide

The lotion compositions of the present invention may comprise niacinamide skin treatment agent as an individual niacinamide or as a combination of niacinamides at a total niacinamide concentration ranging from about 0.01% to about 10% or from about 0.05% to about 5% or from about 0.2% to about 2% by weight of the lotion composition. The niacinamide skin treatment agent provides for skin conditioning benefits as well as providing for increased efficacy of the skin treatment agents in controlling skin disorders.

The niacinamide has the following formula:

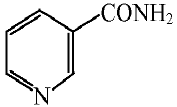

### D. Optional ingredients

One or more optional components known or otherwise effective for use in lotion compositions may be included provided that the optional components are physically and chemically compatible with the essential skin treatment agent and carrier components, or do not otherwise unduly impair product stability, aesthetics, or performance. Such optional components are typically included at concentrations ranging from about 0.001 % to about 20% by weight of the lotion composition, and include materials such as water, skin conditioning agents, perfumes, deodorants, opacifiers, astringents, preservatives, emulsifying agents, film formers, stabilizers, proteins, lecithin, urea, colloidal oatmeal, pH control agents, and particularly, other as below:

### Panthenol

Where included, panthenol typically may comprise from about 0.001% to about 10% or from about 0.005% to about 5% or from about 0.05% to about 1% by weight of the lotion composition. The optional panthenol ingredient provides for skin emolliency benefits that can leave the body feeling smooth, soothing, and soft during and after interaction of the body tissues with the skin treatment agents. The lotion compositions of the present invention may include an individual panthenol compound or a mixture of panthenol compounds.

### Glycerine

Where included, the lotion compositions may comprise the preferred optional glycerine ingredient at concentrations ranging from about 0.01 % to about 10% or from about 0.02% to about 5% or from about 0.05% to about 2% by weight of the lotion composition. The optional glycerine ingredient also provides for skin emolliency benefits such as smooth, soothing, and soft feeling body, as well as being a dispersing agent for the niacinamide skin treatment agent.

### Chamomille

The lotion compositions may comprise the preferred optional chamomile oil at concentrations ranging from about 0.0001 % to about 10%, or from about 0.001 % to about 5% or from about 0.005% to about 2% by weight of the lotion composition. The optional chamomile oil ingredient also provides for skin benefits such as soothing. Chamomile oil is commonly prepared as an oil extract of chamomile flowers. An example of a commercially available chamomile oil include Phytoconcentrol Chamomile which is available from Dragoco Incorporation (Totowa, New Jersey).

### Silk Protein or Silk Amino Acids or Silk Peptides

Silk protein is composed of silk fiber and sericin. The silk protein is produced by species of the *Phylum Arthropoda,* classes *Insecta* and *Arachnida.* Sericin and/or silk amino acids and/or silk peptides are amenable to binding to the skin and hair, forming a resistant, moisturizing, and protective film on the skin/hair. The optional silk ingredient also provides for body benefits such as soothing, moisturizing, and conditioning. The lotion compositions may comprise the preferred optional silk protein or silk amino acids, or mixtures thereof at concentrations ranging from about 0.0001% to about 25% or from about 0.0005% to about 15% or from about 0.001% to about 10% by weight of the lotion composition.

### II. Carrier

The lotion compositions of the present invention may comprise a carrier for the skin treatment agents. The carrier can be included in the lotion compositions as an individual carrier or a combination of carrier ingredients, provided that the total carrier concentration is sufficient to provide transfer and/or migration of the skin treatment agents onto the skin. The carrier can be a liquid, solid, or semisolid carrier material, or a combination of these materials, provided that the resultant carrier forms a homogenous mixture or solution at selected processing temperatures for the resultant carrier system and at processing temperatures for combining the carrier with the skin treatment agents in formulating the lotion compositions herein. Processing temperatures for the carrier system typically range from about 60°C to about 90°C or from about 70°C to about 85°C or from about 70°C to about 80°C.

The lotion compositions of the present invention may typically comprise the carrier at a total carrier concentration ranging from about 60% to about 99.9% or from about 70% to about 98% or from about 80% to about 97% by weight of the lotion composition. Suitable carrier compounds may include petroleum-based hydrocarbons having from about 4 to about 32 carbon atoms, fatty alcohols having from about 12 to about 24 carbon atoms, polysiloxane compounds, fatty acid esters, alkyl ethoxylates, lower alcohols, particularly the lower alcohols having from about 4 to about 6 carbon atoms, low molecular weight glycols and polyols, fatty alcohol ethers having from about 12 to about 28 carbon atoms in their fatty chain, lanolin and its derivatives, glyceride and its derivatives including acetoglycerides and ethoxylated glycerides of C₁₂-C₂₈ fatty acids, and mixtures thereof.

It is believed that a petroleum-based carrier system comprising C₄-C₃₂ hydrocarbons, C₁₂-C₂₂ fatty alcohols, and fumed silica provides a homogeneous mixture of the carrier, skin treatment agents, and any optional ingredients wherein this homogeneous mixture ensures sufficient contact between the skin and skin treatment agents to result in effective prevention and treatment of skin disorders. The fumed silica suitable for inclusion in the preferred petroleum-based carrier system, or with any other carrier described herein, includes colloidal pyrogenic silica pigments which are sold under the Cab-O-Sil^{®} tradename, and which are commercially available from the Cabot Corporation located in Tuscola, Illinois.

In case that the lotion composition is fluid and mobile at ambient temperatures, the lotion composition tends not to remain localized on the body-facing surface 15 of the topsheet 26, but instead may migrate through the topsheet 26 into the interior of the absorbent article, e.g. into the absorbent structure 20. In order to impede the lotion compositions to migrate to other parts of the absorbent article 10, non-limiting examples of suitable immobilizing agents including well-known natural or synthetic waxes may be used.

In a preferred embodiment, the topsheet 26 is hydrophobic or rendered to be hydrophobic, and each lotion stripe 30 is also hydrophobic. Indeed, menstrual fluid, which has a much greater viscosity and much lower fluid flow, both in quantity and time, than urine can be very effectively handled with a hydrophobic topsheet. Whereas urine may simply run off of a hydrophobic topsheet, particularly one that is treated with a hydrophobic lotion, it has been found that such a structure provides for superior benefits in a catamenial pad for menstruating women. Another unexpected benefit is the coating of the skin and hair of the vulvar region during use of a catamenial device that results in cleaner skin and hair of the vulvar region. Yet, another benefit is better fluid acquisition of the fluid due to the transfer of the lotion composition to the skin of the wearer that minimizes fluid transport on the skin and hair of the wearer away from the point of exit.

### Method of Manufacture

The lotion compositions of the present invention may be prepared by any known or otherwise effective technique known in the art and suitable for providing the lotion composition which may comprise the essential ingredients defined herein, e.g. the skin treatment agents. In general, the lotion compositions may be prepared by first making a carrier system comprising suitable carriers such as petrolatum and behenyl alcohol in combination with a fumed silica thickening agent. Next, a mixture comprising the skin treatment agents and any optional ingredients are added to the carrier system at a melt mix temperature of about 80°C. Although the carrier system, skin treatment agents, and any optional ingredients are typically processed at a temperature of about 80°C, these materials can be processed at temperatures ranging from about 60°C to about 90°C or from about 70°C to about 90°C.

The lotion compositions of the present invention are prepared such that the lotion compositions can be applied to the absorbent article 10 to result in safe and effective amounts of the lotion compositions being transferred onto the skin of a wearer of the absorbent article 10.

The lotion compositions of the present invention can be applied to the absorbent articles 10 by any known or otherwise effective technique for distributing a lotion composition onto the absorbent article 10. Nonlimiting examples of methods of applying the lotion compositions onto the absorbent article 10 include spraying, printing (e.g., flexographic printing), coating (e.g., contact slot coating and gravure coating), extrusion, or combinations of these application techniques. The application of the lotion compositions onto the absorbent article 10 facilitates the transfer or migration of the lotion compositions onto the wearer's skin for administration and/or deposition of the lotion compositions, resulting in a safe and effective amount of the lotion compositions being applied for improved prevention and reduction of skin disorders.

The lotion compositions are typically applied to the topsheet 26 of the absorbent article 10 for delivery of the lotion composition onto an external or internal surface of the body. The lotion composition can be applied to other areas of the absorbent article 10 wherein these areas include wings, side panels, the absorbent structure 20, any secondary layer intermediate the absorbent structure 20 and the topsheet 26, or any other region of the absorbent article 10. For instance, the resultant lotion composition is subsequently applied to the topsheet 26 of the absorbent article 10 using a contact lotion depositing station such as a Nordson EP 11-12-02.

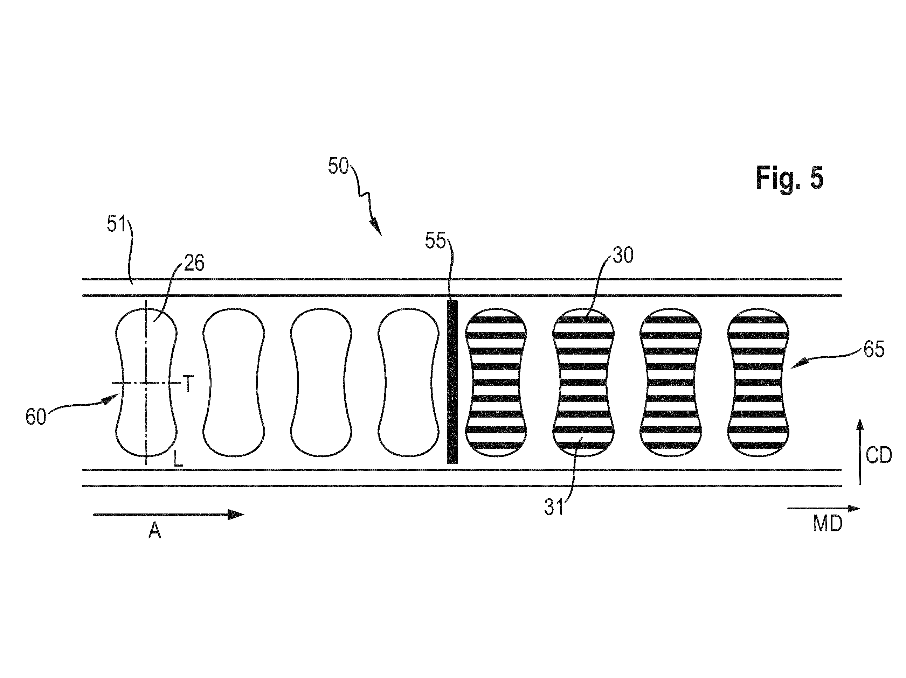

Fig. 5 illustrates a schematic view of the manufacturing process 50. One preferred method involves spraying of the coating on the topsheet 26 before the topsheet 26 is assembled with the other raw materials into a finished absorbent article 10.

The method may involve continuous or intermittent spraying of the lotion composition (or any other methods of applying the lotion composition as set out above) onto the topsheet 26 of the absorbent article 10 during the converting operation. Referring to Fig. 5, conveyor belt 51 advances in the direction shown by the arrow A. Conveyor belt 51 carries a plurality of absorbent articles 60 having no lotion composition in the topsheet 26 towards a lotion depositing station 55 where the topsheet 26 is sprayed with the lotion composition. The lotion composition may be applied at a temperature where it is suitably fluid enough, e.g. from about 40°C to about 80°C.

A plurality of at least 2 lotion stripes 30 having a lotion composition is deposited. The lotion stripes are separated by one or more lotion free areas 31. In Fig. 5, the plurality of lotion stripes 30 is oriented parallel with the transverse centerline of the absorbent article 10. Indeed, the plurality of lotion stripes 30 is oriented parallel with the machine direction of the equipment. In conventional manufacturing processes, the longitudinal centerline of each absorbent article 10 is parallel with the machine direction of the equipment. The application of lotion stripes 30 in cross direction would require more sophisticated and precise equipment (e.g. printing equipment) whereas in this method, the transverse centerline is parallel with the machine direction of the equipment. Hence, the standard lotion depositing station 55 allows obtaining the desired absorbent articles 10 easily.

The lotion station may be modified in order to manufacture the different embodiments as described above:
The lotion depositing station 55 may deposit in a continuous fashion the lotion composition on the plurality of absorbent articles 10, for an easier manufacturing, i.e. thus obtaining absorbent articles 10 like the ones shown in Fig. 3 and 4.

Alternatively, the lotion depositing station 55 may deposit the lotion composition onto a portion of the width of the absorbent article 10 generally centered around and along the longitudinal centerline, thus obtaining absorbent articles 10 like the ones shown in Fig. 2. This allows to reduce the amount of the lotion composition deposited onto the topsheet 26, focusing the application of the lotion composition toward the central portions of the absorbent article 10. However, in this case, the lotion depositing station 55 must stop and start again at the precise positions in order to obtain the desired result.

After leaving the lotion depositing station 55, nonlotioned absorbent article 60 becomes lotioned absorbent article 65 having a lotioned topsheet. The amount of lotion composition transferred to topsheet is controlled by: (1) the rate at which the molten lotion composition is sprayed from the lotion depositing station 55; and/or (2) the speed at which conveyor belt travels under the lotion depositing station 55.

## Claims

1. An absorbent article comprising:
a backsheet;
a topsheet joined to the backsheet, the topsheet defining a body-facing surface;
a transverse centerline;
a lotion composition deposited on at least a portion of the body-facing surface of the topsheet;
**characterized in that**
the lotion composition is applied to the body-facing surface of the topsheet in the form of a pattern of a plurality of at least two lotion stripes, the lotion stripes being separated by one or more lotion free areas;
wherein each lotion free area is defined by the region between two neighboring lotion stripes;
wherein the plurality of lotion stripes is oriented parallel with the transverse centerline of the absorbent article;
wherein the ratio between the width of each lotion free area and the width of any of the neighboring lotion stripes of each lotion free area is from 0.5 to 50.

2. The absorbent article of claim 1 is a feminine care absorbent article wherein the feminine absorbent article is selected from the group consisting of a sanitary napkin, an interlabial absorbent article and a pantiliner.

3. The absorbent article of any of the preceding claims further comprises an absorbent structure positioned between the topsheet and the backsheet.

4. The absorbent article of any of the preceding claims wherein each lotion stripe of the plurality of lotion stripes is extending parallel with the transverse centerline over at least a portion of the absorbent article.

5. The absorbent article of any of the preceding claims wherein each lotion stripe of the plurality of lotion stripes is extending parallel with the transverse centerline over the full width of the absorbent article.

6. The absorbent article of any of the preceding claims wherein the width of each lotion free area is from 0.25 mm to 80 mm.

7. The absorbent article of any of the preceding claims wherein each lotion stripe comprises 5 g/m² to 30 g/m² of the lotion composition.

8. The absorbent article of any of the preceding claims wherein the lotion stripes are of equal width whereas the lotion free areas separating the lotion stripes are not of equal width.

9. The absorbent article of any of the preceding claims in the form of a pattern of a plurality of at least four lotion stripes being separated by at least three lotion free areas wherein the ratio between the width of the lotion free area and the width of any of the neighboring lotion stripes of each lotion free area is increasing from a transversal edge of the absorbent article towards the transversal centerline of the article.

10. The absorbent article of any of the preceding claims wherein the lotion composition is transferable to the wearer's skin.

11. Method for applying the lotion composition on the body-facing surface of the absorbent article according to claim 1 comprising the following steps:
(a) advancing in a production line, a plurality of absorbent articles towards a lotion depositing station, wherein each absorbent article has its transversal centerline parallel to the machine direction of the product manufacturing equipment;
(b) depositing a plurality of at least 2 lotion stripes having a lotion composition, the lotion stripes being separated by one or more lotion free areas wherein the plurality of lotion stripes is oriented parallel with the transverse centerline of the absorbent article, wherein the ratio between the width of each lotion free area and the width of any of the neighboring lotion stripes of each lotion free area is from 0.5 to 50.

12. Method of claim 11 wherein the lotion composition is deposited to the body-facing surface of the topsheet of the absorbent article via spraying, printing, gravure coating, extrusion and any combination thereof.

13. Method of claims 11 and 12 wherein the depositing of a plurality of at least 2 lotion stripes having the lotion composition is continuous and cover the entire width of the absorbent article.

14. Method of claims 11 and 12 wherein the depositing of a plurality of at least 2 lotion stripes having the lotion composition is intermittent and cover only a portion of the width of the absorbent article.
